# EUROPÄISCHE PATENTSCHRIFT

(11) **EP 1 133 973 B1**
(45) Veröffentlichungstag und Bekanntmachung des Hinweises auf die Patenterteilung: **19.03.2003**
(21) Anmeldenummer: 00127532.0
(22) Anmeldetag: 15.12.2000
(51) Int. Cl.: A61K 7/075

(54) **Haarpflegemulsion**
Hair care emulsion
Emulsion pour le soin des cheveux

(30) Priorität: 19.01.2000 DE 10002107; 02.12.2000 DE 10060056
(43) Veröffentlichungstag der Anmeldung: 19.09.2001
(73) Patentinhaber: KPSS-Kao Professional Salon Services GmbH, 64297 Darmstadt (DE)
(72) Erfinder: Heinz, Dieter, 65462 Gustavsburg (DE)

(56) Entgegenhaltungen:
- EP-A- 0 186 025
- DE-A- 4 009 617
- DE-A- 19 722 043
- DE-C- 19 815 341
- FR-A- 2 740 331
- US-A- 5 362 484
- US-A- 5 908 618
- PATENT ABSTRACTS OF JAPAN vol. 1998, no. 11, 30. September 1998 (1998-09-30) & JP 10 175824 A (LION CORP)

## Beschreibung

Die vorliegende Erfindung betrifft ein Haarpflegemittel in Form einer Wasser-in-Öl-Emulsion mit verbesserten haarkonditionierenden Eigenschaften.

Es ist seit Jahrzehnten bekannt, Haarkonditioniermittel der verschiedensten Art zu benutzen. Sie enthalten in der Regel kationische Wirkstoffe, insbesondere quaternäre langkettige Ammoniumverbindungen, und/oder Polymere mit quaternären Stickstoffatomen und werden insbesondere als Lösungen, Öl-in-Wasser-Emulsionen, Gele oder Lotionen eingesetzt, die auch als Aerosole oder Pumpsprays zum Einsatz gelangen können.

Entsprechende Zusammensetzungen, die entweder nach der Applikation im Haar verbleiben oder aber auch ausgewaschen werden können, sind beispielsweise in der Monographie von K. Schrader, Grundlagen und Rezepturen der Kosmetika, 2. Aufl., (Hüthig Buch Verlag, 1989) auf den Seiten 722 bis 781 unter dem Stichwort "Haarnachbehandlungsmittel" beschrieben.
Trotz ihrer Vielfalt sind diese Produkte hinsichtlich ihrer anwendungstechnischen Eigenschaften noch immer verbesserungsfähig.

Es wurde nunmehr festgestellt, und das ist Gegenstand der vorliegenden Erfindung, daß ein optimales konditionierendes Haarnachbehandlungsmittel aus einer Wasser-in-Öl-Emulsion besteht, die in der wäßrigen Phase mindestens ein wasserlösliches Polymer und in der Fett- bzw. Ölphase mindestens ein Pflanzenproteinhydrolysat, dessen Klarlöslichkeit im Wasser bei 20°C weniger als 0,5g/100 ml H₂O, vorzugsweise weniger als 0,3g/100 ml H₂O beträgt, enthält.

Dieses Produkt verleiht dem Haar nicht nur Glanz, Fülle und Volumen, einen vollen Griff, Spannkraft und verbesserte Naß- und Trockenkämmbarkeit, sondern auch eine lang anhaltende Festigungswirkung, die durch das Verhältnis Polymer/Proteinhydrolysat regulierbar ist, sondern weist auch keinerlei sonst bei solchen Produkten häufig auftretende Klebrigkeit oder Hygroskopizität auf dem Haar auf.

Das Pflanzenproteinhydrolysat, dessen Klarlöslichkeit im Wasser bei 20°C weniger als 0,5g/100 ml H₂O, vorzugsweise weniger als 0,3g/100 ml H₂O beträgt, verbleibt dabei offensichtlich nahezu ausschließlich in der Öl- bzw. Fettphase der W/O-Emulsion und diffundiert nicht in die Wasserphase derselben.

Das Molekulargewicht dieses Pflanzenproteinhydrolysats liegt vorzugsweise zwischen etwa 10 000 und etwa 500 000, insbesondere etwa 15 000 bis 400 000, besonders bevorzugt etwa 25 000 bis 250 000 g/Mol.

Geeignete Planzenproteinhydrolysate sind beispielsweise solche aus Sojabohnenprotein, Mandelprotein, Weizenprotein und sonstigen Getreideproteinen, Kartoffelprotein und insbesondere aus Erbsenprotein, Pisum sativum.

Die geeignete Menge des Pflanzenproteinhydrolysats liegt bei etwa 0.1 bis 5, insbesondere etwa 0,25 bis etwa 2,5 Gew.-%, berechnet auf die gesamte W/O-Emulsion.

Geeignete wasserlösliche Polymere, die in der Wasserphase der erfindungsgemäßen W/O-Emulsion vorliegen, sind ebenfalls grundsätzlich seit langem bekannt.

Ihre Menge beträgt vorzugsweise etwa 0,25 bis 10, insbesondere etwa 0,5 bis etwa 7,5, vor allem etwa 1 bis 5 Gew.-%, berechnet auf die Gesamtzusammensetzung der Emulsion.

Bevorzugt sind nichtionische Polymere wie wasserlösliche Vinylpyrrolidon-Polymere, z.B. Vinylpyrrolidon-Mono- oder Copolymerisate, insbesondere mit Vinylacetat.

Geeignete Vinylpyrrolidon-Polymere sind z.B. die unter dem Handelsnamen "Luviskol®" bekannten Produkte, beispielsweise die Homopolymerisate "Luviskol® K 30, K 60 und K 90" sowie die wasserlöslichen Copolymerisate aus Vinylpyrrolidon und Vinylacetat, die unter dem Handelsnamen "Luviskol® VA 55 bzw. VA 64" von der BASF AG vertrieben werden.

Weitere geeignete nichtionische Polymere sind Vinylpyrrolidon/Vinylacetat/Vinylpropionat-Copolymere wie "Luviskol® VAP 343", Vinylpyrrolidon/(Meth)-Acrylsäureester-Copolymere sowie Chitosan-Derivate.

Anstelle oder zusätzlich zu den nichtionischen Polymeren können als konditionierende Polymere insbesondere kationische und/oder auch anionische undloder amphotere Polymere in den genannten Mengen eingesetzt werden.

Bevorzugte konditionierende kationische Polymere sind die altbekannten quaternären Cellulosederivate des Typs "Polymer JR" sowie quaternisierte Homo- und Copolymere des Dimethyldiallylammoniumchlorids, wie sie unter dem Handelsnamen "Merquat®" im Handel sind, quaternäre Vinylpyrrolidon-Copolymere, insbesondere mit Dialkylaminoalkyl(meth)acrylaten, wie sie unter dem Namen "Gafquat®" bekannt sind, Copolymerisate aus Vinylpyrrolidon und Vinylimidazoliniummethochlorid, die unter dem Handelsnamen "Luviquat®" angeboten werden, Polyamino-Polyamid-Derivate, beispielsweise Copolymere von Adipinsäure-Dimethylaminohydroxypropyldiethylentriamin, wie sie unter dem Namen "Cartaretine® F" vertrieben werden, sowie auch bisquaternäre langkettige Ammoniumverbindungen der in der US-PS 4 157 388 beschriebenen Harnstoff-Struktur, die unter dem Handelsnamen "Mirapol® A 15" erhältlich sind.
Verwiesen wird in diesem Zusammenhang auch auf die in den DE-OSen 25 21 960, 28 11 010, 30 44 738 und 32 17 059 genannten kationaktiven Polymeren sowie die in der EP-A 337 354 auf den Seiten 3 bis 7 beschriebenen Produkte. Es können auch Mischungen verschiedener kationischer Polymerer eingesetzt werden.
Zu den kationischen Polymeren zählen auch die in der EP-A 524 612 und der EP-A 640 643 beschriebenen Quaternisierungsprodukte aus Pfropfpolymerisaten von Organopolysiloxanen und Polyethyloxazolinen.

Als amphotere Polymere, die allein oder im Gemisch mit mindestens einem weiteren kationischen, nichtionischen oder anionischen Polymeren zum Einsatz gelangen, seien insbesondere Copolymerisate aus N-Octylacrylamid, (Meth)Acrylsäure und tert.-Butylaminoethylmethacrylat vom Typ "Amphomer®"; Copolymerisate aus Methacryloylethylbetain und Alkylmethacrylaten vom Typ "Yukaformer®", z. B. das Butylmethacrylat-Copolymere "Yukaformer® Am75"; Copolymerisate aus Carboxylgruppen und Sulfongruppen enthaltenden Monomeren, z. B. (Meth)Acrylsäure und Itaconsäure, mit basische Gruppen, insbesondere Aminogruppen, enthaltenden Monomeren wie Mono- bzw. Dialkylaminoalkyl(meth)acrylaten bzw. Mono- bzw. Dialkylaminoalkyl(meth)acrylamiden; Copolymere aus N-Octylacrylamid, Methylmethacrylat, Hydroxypropylmethacrylat, N-tert.-Butylaminoethylmethacrylat und Acrylsäure sowie die aus der US-A 3,927,199 bekannten Copolymeren genannt.

Geeignete anionische Polymere sind Vinylalkylether-, insbesondere Methylvinylether/Maleinsäure-Copolymere, die durch Hydrolyse von Vinylether/Maleinsäureanhydrid-Copolymeren entstehen und unter der Handelsbezeichnung "Gantrez® AN oder ES" vertrieben werden. Diese Polymere können auch teilverestert sein, beispielsweise "Gantrez® ES 225", der Ethylester eines Ethylvinylethers/Maleinsäure-Copolymers, oder der Butyl- oder Isobutylester desselben.

Weitere geeignete anionische Polymere sind insbesondere Vinylacetat/Crotonsäure- oder Vinylacetat/Vinylneodecanoat/Crotonsäure-Copolymere des Typs "Resyn®"; Natriumacrylat/Vinylalkohol-Copolymere des Typs "Hydagen® F", Natriumpolystyrolsulfonat, z.B. "Flexan® 130"; Ethylacrylat/Acrylsäure/N-tert.-Butylacrylamid-Copolymere des Typs "Ultrahold®"; Vinylpyrrolidon/Vinylacetat/Itaconsäure-Copolymere, Acrylsäure/Acrylamid-Copolymere bzw. Natriumsalze derselben vom Typ "Reten®"; etc.

Die erfindungsgemäßen Mittel können neben den beiden essentiellen zusätzlich auch noch weitere konditionierende wasserlösliche Eiweißhydrolysate und Polypeptide, z.B., Keratinhydrolysate, Kollagenhydrolysate vom Typ "Nutrilan®" oder Elastinhydrolysate sowie insbesondere auch pflanzliche, gegebenenfalls kationisierte Eiweißhydrolysate, z.B. "Gluadin^{R}", enthalten.

Als solche seien beispielhaft Komplexbildner, Farbstoffe, Konservierungsmittel, pH-Regler, Viskositätsregler wie anorganische Salze, soweit sie nicht ohnehin in den Tensid-Ausgangsmischungen enthalten sind, Duftstoffe, Perlglanzmittel, Verdickungsmittel, Feuchthaltemittel, pflanzliche und tierische Öle wie Jojobaöl, Fettsäureester wie z.B. Isopropylmyristat, Ethylpalmitat, Lecithin und dessen Derivate, etc. genannt.
Schließlich können auch noch bekannte Polysiloxane als konditionierende Mittel in den erfindungsgemäßen Haarpflegeemulsionen, vorzugsweise in der Öl- bzw. Fettphase, mitverwendet werden. Deren bevorzugter Anteil liegt dabei etwa zwischen 0,5 und etwa 5, insbesondere 1 bis 3 Gew.-% der Gesamtzusammensetzung.

Geeignet sind sowohl leichtflüchtige als auch schwerflüchtige cyclische oder lineare Polysiloxane, beispielsweise die unter den Trivialnamen "Dimethicone" bzw. "Phenyldimethicone" sowie "Cyclomethicone" bekannten Silikonöle, sowie beispielsweise auch die in der EP-A 398 177 beschriebenen Silikonderivate, die dort in Kombination mit Alkylpolyglucosiden in flüssigen Detergens-Zusammensetzungen eingesetzt werden.

Ein weiterer bevorzugter Bestandteil in den erfindungsgemäßen Mitteln sind Pflanzenextrakte in einer Menge von etwa 0,01 bis etwa 10, vorzugsweise 0,1 bis 7,5, insbesondere 0,5 bis 5 Gew.-%, berechnet als Trockenrückstand desselben auf die Gesamtmenge des Mittels, die vorzugsweise in der wäßrigen Phase der erfindungsgemäßen W/O-Emulsion vorliegen.

Geeignete, an sich bekannte wäßrige (z.B. Wasserdampf-destillierte), alkoholische oder wäßrig-alkoholische Pflanzenextrakte sind insbesondere Extrakte von Blättern, Früchten, Blüten, Wurzeln, Rinden oder Stämmen von Aloe, Ananas, Artischoken, Arnika, Avocado, Baldrian, Bambus, Bilsenkraut, Birke, Brennesseln, Echinacea, Efeu, Engelwurz, Enzian, Farnen, Fichtennadeln, Gänsefingerkraut, Ginseng, Ginster, Hafer, Hagebutten, Hamamelis, Heublumen, Holunder, Hopfen, Huflattich, Johannisbeeren, Kamillen, Karotten, Kastanien, Klee, Klettenwurzeln, Kokosnuß, Korn, Lindenblüten, Maiglöckchen, Meeralgen, Melisse, Mistel, Passionsblumen, Ratanhia, Ringelblumen Rosmarin, Roßkastanien, Rotdorn, Salbei, Schachtelhalm, Scharfgarbe, Schlüsselblumen, Taubnesseln, Thymian, Walnüssen, Weinblättern, Weißdorn, etc.

Geeignete Handelsprodukte sind beispielsweise die verschiedenen "Extrapone^{R}", "Herbasol^{R}", "Sedaplant^{R}" und "Hexaplant^{R}". Extrakte und deren Herstellung sind auch in "Hagers Handbuch der pharmazeutischen Praxis", 4. Aufl., beschrieben.

Die erfindungsgemäßen Nachbehandlungsmittel können vorzugsweise noch mindestens ein kationisches Tensid, wie eine quaternäre Ammoniumverbindung, die auch konditionierend wirkt, enthalten.
Geeignete langkettige quaternäre Ammoniumverbindungen, die allein oder im Gemisch miteinander eingesetzt werden können, sind beispielsweise Cetyltrimethylammoniumchlorid, Dimethyldicetylammoniumchlorid, Trimethylcetylammoniumbromid, Stearyltrimethylammoniumchlorid, Dimethylstearylbenzylammoniumchlorid, Benzyltetradecyldimethylammoniumchlorid Dimethyldihydriertes-Talgammoniumchlorid, Laurylpyridiniumchlorid, Lauryldimethylbenzylammoniumchlorid, Behenyltrimethylammoniumchlorid, Lauryltrimethylammoniumchlorid, Tri(oligooxyethl)alkylammoniumphophat, Cetylpyridiniumchlorid, etc. Gut geeignet sind auch die in der EP-A 472 107 geoffenbarten quaternären Ammoniumsalze.
Im Prinzip sind alle quaternären Ammoniumverbindungen, wie sie im jeweils gültigen "CTFA International Cosmetic Ingredient Dictionary" unter dem Trivialnamen "Quaternium" aufgeführt sind, einsetzbar.
Ihr Anteil liegt vorzugsweise bei etwa 0,5 bis 10, insbesondere etwa 1 bis 7,5 Gew.-%, berechnet auf die Gesamtzusammensetzung.
Besonders geeignete langkettige Ammoniumverbindungen sind Esterquats der allgemeinen Formel (I) 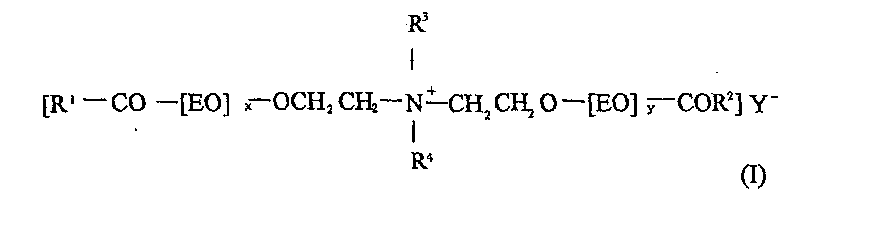 in der R¹ und R² für eine gegebenenfalls hydroxysubstituierte C₈-C₂₂-Alkyl- oder Alkenylgruppe, R³ und R⁴ für eine C₁-C₃-Alkylgruppe oder eine Gruppe -CH₂-CH₂-O-[EO]_{z}H sowie x,y und z für 0 bis 5 und Y⁻ für ein Anion stehen.
Eine besonders bevorzugte Verbindung der Formel I ist im Rahmen der Erfindung eine solche, in der die Reste R¹ und R² jeweils eine Oleylgruppe oder eine C₁₂-C₁₈-Alkylgruppe, der Rest R³ eine Methylgruppe und der Rest R⁴ eine Gruppe -CH₂-CH₂-O-[EO]_{z}-H bedeuten.
Das Anion Y⁻ ist vorzugsweise ein Halogenid wie Cl⁻ oder Br⁻, ein niederes Alkylsulfat, z.B. Methosulfat und Ethosulfat, oder ein Alkylphosphat, jedoch können selbstverständlich auch andere Reste eingesetzt werden.
Diese Verbindungen sind an sich bekannt und beispielsweise unter den Handelsnamen "Schercoquat^{A}", "Dehyquart^{R}F30" und "Tetranyl^{R}" im Handel.
Der Einsatz dieser Verbindungen, sogenannter "Esterquats", in Haarpflegemitteln ist an sich bekannt und beispielsweise in der WO-A 93/10748, der WO-A 92/06899 und der WO-A 94/16677 beschrieben, wo sich jedoch keinerlei Hinweise auf die erfindungsgemäße Kombination und deren vorteilhafte Eigenschaften finden.

Geeignet sind auch Amidoquats der allgemeinen Formel (II) 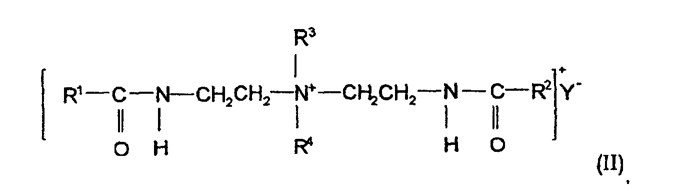 in der R¹ und R² jeweils für eine gegebenenfalls hydroxysubstituierte C₈-C₂₂-Alkyl- oder Alkenylgruppe, R³ und R⁴ für eine C₁-C₃-Alkylgruppe oder eine Gruppe-CH₂-CH₂-O-[EO]ₓ-H, sowie x für 0 bis 5, Y⁻ für ein Anion stehen.

Die erfindungsgemäßen Nachbehandlungsmittel enthalten in der Fettphase Öle und Fette.
Geeignete Fette und Öle, zu denen auch Wachse zählen, sind insbesondere natürliche Öle wie Avocadoöl, Cocosöl, Palmöl, Sesamöl, Erdnußöl, Spermöl, Sonnenblumenöl, Mandelöl, Pfirsichkernöl, Weizenkeimöl, Macadamianußöl, Nachtkerzenöl, Jojobaöl, Ricinusöl, oder auch Oliven- bzw. Sojaöl, Lanolin und dessen Derivate, ebenso Mineralöle wie Paraffinöl und Vaseline.
Synthetische Öle und Wachse sind beispielsweise Silikonöle, Polyethylenglykole, etc.

Weitere geeignete hydrophobe Komponenten sind insbesondere Fettsäureester wie Isopropylmyristat, -palmitat, -stearat und -isostearat, Oleyloleat, Isocetylstearat, Hexyllaurat, Dibutyladipat, Dioctyladipat, Myristiylmyristat, Oleylerucat, Polyethylenglykol- und Polyglycerylfettsäureester wie PEG-7-glycerylcocoat, Cetylpalmitat, etc.
Diese hydrophoben Komponenten sind in den erfindungsgemäßen Nachbehandlungs-Zusammensetzungen vorzugsweise in einer Gesamtmenge von etwa 5 bis etwa 50, insbesondere etwa 7,5 bis 35, vor allem etwa 10 bis 25 Gew.-%, berechnet auf die Gesamtzusammensetzung der Emulsion, enthalten.

Die erfindungsgemäßen Nachbehandlungsmittel können auch langkettige Fettsäuren enthalten.
Als Fettsäuren werden bevorzugt solche mit 10 bis 24, insbesondere 12 bis 22 Kohlenstoffatomen in einer Menge von etwa 0,5 bis 15 Gew.-%, insbesondere 1 bis 10 Gew.-%, bezogen auf die Gesamtzusammensetzung, verwendet. Besonders geeignet sind Behensäure und Stearinsäure; jedoch können auch andere Fettsäuren wie beispielsweise Myristinsäure, Palmitinsäure oder Ölsäure oder auch Gemische natürlicher oder synthetischer Fettsäuren wie Kokosfettsäure eingesetzt werden.

Ein weiterer bevorzugter Bestandteil in Nachbehandlungsmitteln ist Harnstoff, vorzugsweise in einer Menge von etwa 1 bis 10, insbesondere etwa 2,5 bis 7,5 Gew.-% des erfindungsgemäßen Mittels.

Das erfindungsgemäße Haarbehandlungsmittel kann als weiteren Bestandteil noch mindestens eine Verbindung, ausgewählt aus der Gruppe 1-Methoxypropanol(-2), 1-Ethoxypropanol(-2), Diethylenglykolmonomethyl- oder ethylether, Dipropylenglykolmonomethyl- oder -ethylether, Benzylalkohol, Benzyloxyethanol, Phenylethylalkohol, Phenoxyethanol und/oder Zimtalkohol, vorzugsweise in einer Menge von 0,5 bis 25, insbesondere 1 bis 20, vor allem 2,5 bis 15 Gew.-%, berechnet auf die Gesamtzusammensetzung des Mittels, enthalten.
Bevorzugte Verbindungen aus dieser Gruppe sind Ethoxydiglykol und Benzyloxyethanol.
Dabei gilt grundsätzlich, daß wasserlösliche Ingredientien in der Wasserphase und wasserunlösliche Ingredientien in der Öl- bzw. Fettphase der erfindungsgemäßen W/O-Emulsion enthalten sind, wobei natürlich auch Grenzfälle auftreten können.

Die erfindungsgemäßen Wasser-in-Öl-Emulsionen enthalten mindestens einen geeigneten W/O-Emulgator, vorzugsweise mindestens ein nichtionische Tensid,
Diese sind grundsätzlich bekannt und beispielsweise in der Monographie von K. Schrader, l.c., S 387 bis 525, ausführlich beschrieben, auf die, zur Vermeidung von Wiederholungen, hier ausdrücklich Bezug genommen wird.
Üblicherweise werden Emulgatorgemische eingesetzt, z.B. aus (Poly-) Glycerinfettsäureestern mit Fettalkoholpolyglykolethern.
Auch die Mitverwendung von Coemulgatoren wie z.B. höheren Fettalkoholen ist üblich und zweckmäßig.
Bezüglich der Einzelheiten wird auf den bekannten Stand der Technik verwiesen, der auch die mögliche Mitverwendung von anorganischen und organischen Hydrokolloiden, Konsistenzreglern, Stabilisatoren etc. ausführlich beschreibt (vgl. Schrader, I.c., S. 407 bis 411).
Der Anteil an W/O-Emulgatoren in der Gesamtzusammensetzung ist von deren Struktur und HLB-Wert abhängig und liegt bei etwa 2,5 bis 25, insbesondere etwa 5 bis 15 Gew.-%, je nach Art der vorhandenen weiteren Bestandteile, insbesondere der Öle, Fette und Wachse und deren mögliche oberflächenaktiven Eigenschaften.
Gleiches gilt für das Verhältnis Wasserphase zu Öl- bzw. Fettphase in der W/O-Emulsion, die bei etwa 10 zu 90 bis 90 zu 10 betragen kann vorzugsweise jedoch bis etwa 20:80 bis 80:20, insbesondere bei etwa 30:70 bis 70:30 liegt.

Die Herstellung solcher W/O-Emulsionen ist im Prinzip ebenfalls bekannt; sie erfolgt üblicherweise durch Aufschmelzen der Fettphase bei erhöhter Temperatur und anschließend dem Zusatz der Wasserphase unter Rühren, Abkühlen und Homogenisieren.
Unter bestimmten Bedingungen und mit bestimmten Zusammensetzungen ist aber auch eine Kaltemulgierung möglich.

Gemäß einer bevorzugten Ausführungsform läßt sich die Wirkung einer solchen Haarpflegeemulsion noch verbessern, wenn man dieser als Emulgator ein Di-C₁₂-C₁₈-acyl-polyglyceryl-2-10 Dimerdioleat, oder -dilinoteat, insbesondere ein -polygyceryl-3-dimerlinoleat, und/oder ein C₈-C₂₀-Alkyl Dimethicone Copolyol der allgemeinen Formel 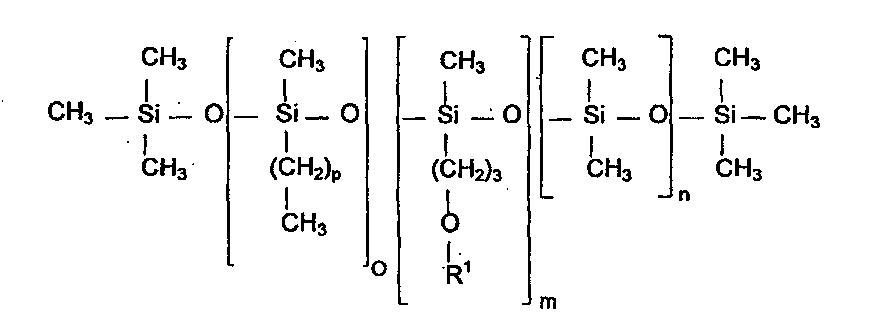 worin R¹ eine Polyethergruppe mit 1-60 Ethylenoxid- und/oder Propylenoxideinheiten, m, n und o je eine Zahl von 1 bis 500, und p eine Zahl von 7 bis 19 Kohlenstoffatomen bedeuten, insbesondere Cetyl oder Lauryl Dimethicone Copolyol, zusetzt.

Ihre Menge beträgt vorzugsweise etwa 0,5 bis 25, insbesondere etwa 2,5 bis etwa 20, vor allem etwa 5 bis 15 Gew.-%, berechnet auf die Gesamtzusammensetzung der Emulsion.
Durch den Zusatz dieser Emulgatoren wird nicht nur die Einarbeitbarkeit des Pflanzenproteinhydrolysats, dessen Klarlöslichkeit im Wasser bei 20°C weniger als 0,5g/100 ml H₂O, vorzugsweise weniger als 0,3g/100 ml H₂O beträgt, verbessert, sondern auch die haarkonditionierenden Eigenschaften wie Glanz und weicher Griff.

Durch die folgenden Beispiele wird die Erfindung näher erläutert.

### Beispiel 1

Es wurde eine W/O-Emulsion durch Schmelzen der Fett- und Ölphase bei 70°C und anschließendem Zugeben der Wasserphase unter Rühren bis zur Erzielung einer homogenen Zusammensetzung hergestellt.

| **Fett- und Ölphase:** | |
|---|---|
| | |
| Erbsenproteinhydrolysat (aus Pisum sativum) | 0,5 (Gew.%) |
| Gesamtproteingehalt ~ 90% (weniger als 0,2 Gew.-% in H₂O bei 20°C löslich) | |
| W/O-Emulgator (PEG-30-dipolyhydroxystearat) | 3,0 |
| Capryl-/Caprinsäuretriglycerid | 4,0 |
| PCL-Liquid | 4,0 |
| Paraffinöl | 2,0 |
| Vaseline | 4,0 |

| **Wasserphase:** | |
|---|---|
| | |
| 1,2-Propandiol | 2,0 (Gew.%) |
| MgSO₄ x 7H₂O | 0,7 |
| Glycerin | 4,0 |
| Vinylpyrrolidon/Vinylacetat-Copolymer | 2,0 |
| Wasser ad | 100,0 |

Diese W/O-Emulsion blieb bei sechsmonatiger Lagerung bei 40°C stabil.
Im Halbseitenversuch wurde das Produkt in die eine Hälfte von frisch shampoonierten handtuchtrockenem Haar einmassiert, die andere Hälfte wurde in gleicher Weise mit einem identischen Produkt (Beispiel 1x) behandelt, das jedoch kein Erbsenproteinhydrolysat enthielt.

Nach 30 Minuten wurden beide Hälften von einem Friseur im Blindtest beurteilt und entsprechend einer Besser-Gleich-Methode eingestuft.

Das Ergebnis zeigte eine eindeutige Präferenz zugunsten der erfindungsgemäßen Zusammensetzung nach Beispiel 1:

| ***Eigenschaften*** | ***Beispiel 1 besser*** | ***Gleich*** | ***Beispiel 1 X besser*** |
|---|---|---|---|
| **Verteilbarkeit des Produkts** | 6 | 2 | 2 |
| **Trockengeschwindigkeit im** | 4 | 4 | 2 |
| **Haar** | | | |
| **Glanz des Haares** | 6 | 4 | 0 |
| **Festigung des Haares** | 8 | 2 | 0 |
| **Volumen, Griff des Haares** | 8 | 2 | 0 |
| **Klebrigkeit des Haares** | 10 | 0 | 0 |
| **Aussehen (Fettigkeit) des Haares** | 9 | 1 | 0 |
| **Lockerheit des Haares** | 8 | 2 | 0 |
| **Gesamtbeurteilung** | 9 | 1 | 0 |

### Beispiel 2

Eine W/O-Emulsion wurde analog der in Beispiel 1 beschriebenen Verfahrensweise hergestellt.

| **Fett- und Ölphase:** | |
|---|---|
| | |
| PEG-30-dipolyhdroxystearat | 2,5 (Gew.%) |
| PEG-7-hydriertes Ricinusöl | 2,5 |
| Isohexadecan | 6,0 |
| Capryl-/Caprinsäuretriglycerid | 4,0 |
| Isopropylmyristat | 4,0 |
| Erbsenproteinhydrolysat (analog Beispiel 1) | 1,0 |

| **Wasserphase:** | |
|---|---|
| | |
| 1,2-Propandiol | 2,0 (Gew.%) |
| MgSO₄ x 7H₂O | 0,7 |
| Glycerin | 4,0 |
| Isobutylen/Maleinsäure-Copolymer (Aquaflex^{R}FX64) | 5,0 |
| Wasser ad | 100,0 |

Diese W/O-Emulsion wies ähnlich gute Eigenschaften wie diejenige nach Beispiel 1 auf.

### Beispiel 3

Analog dem in Beispiel 1 beschriebenen Verfahren wurde eine W/O-Emulsion der folgenden Zusammensetzung hergestellt:

| **Fett- bzw. Ölphase:** | |
|---|---|
| | |
| Kartoffelproteinhydrolysat (Löslichkeit in 100 ml H₂O bei 20°C:< 0,25 g) | 0,5 (Gew.%) |
| Erbsenproteinhydrolysat (Löslichkeit in 100 ml H₂O bei 20°C:< 0,3 g) | 0,5 |
| PEG-30-dipolyhydroxystearat | 2,0 |
| Neo-PCL selbstemulgierend W/O | 2,0 |
| Triglycerindiisostearat | 1,5 |
| Isostearinsäurediethanolamid | 1,5 |
| 2-Octyldodecanol | 2,0 |
| Vaseline | 12,0 |
| Capryl-/Caprinsäuretriglycerid | 4,0 |
| Isopropylstearat | 3,0 |

| **Wasserphase:** | |
|---|---|
| | |
| 1,2-Propandiol | 3,0 (Gew.%) |
| MgSO₄ x 7H₂O | 0,7 |
| Glycerin | 3,0 |
| N-Octylacrylamid/Acrylsäure | 2,5 |
| N-tert. Butylaminoethylmethacrylat-Terpolymer (Amphomer^{R}) | 1,0 |
| Polyvinylpyrrolidon | 0,5 |
| Aminomethylpropandiol | q.s. |
| Konservierungsmittel | q.s. |
| Wasser ad | 100,0 |

Es wurde ein stabiles Produkt mit ausgezeichneten haarkonditionierenden Eigenschaften erhalten.

### Beispiel 4

Es wurde eine W/O-Emulsion durch Schmelzen der Fett- und Ölphase bei 70°C und anschließendem Zugeben der Wasserphase unter Rühren bis zur Erzielung einer homogenen Zusammensetzung hergestellt.

| **Fett- und Ölphase:** | |
|---|---|
| | |
| Erbsenproteinhydrolysat (aus Pisum sativum) | 0,5 (Gew.%) |
| Gesamtproteingehalt ∼ 90% (weniger als 0,2 Gew.-% in H₂O bei 20°C löslich) | |
| Diisostearoylpolyglyceryl-3 Dimerdilinoleat | 3,0 |
| Capryl-/Caprinsäuretriglycerid | 4,0 |
| PCL-Liquid | 4,0 |
| Paraffinöl | 2,0 |
| Vaseline | 4,0 |

| **Wasserphase:** | |
|---|---|
| | |
| 1,2-Propandiol | 2,0 (Gew.%) |
| MgSO₄ x 7H₂O | 0,7 |
| Glycerin | 4,0 |
| Vinylpyrrolidon/Vinylacetat-Copolymer | 2,0 |
| Wasser ad | 100,0 |

Diese W/O-Emulsion blieb bei sechsmonatiger Lagerung bei 40°C stabil.

### Beispiel 5

Eine W/O-Emulsion wurde analog der in Beispiel 4 beschriebenen Verfahrensweise hergestellt.

| **Fett- und Ölphase:** | |
|---|---|
| | |
| Cetyl Dimethicone Copolyol (Abil® EM 90) | 2,5 (Gew.%) |
| PEG-7-hydriertes Ricinusöl | 2,5 |
| Isohexadecan | 6,0 |
| Capryl-/Caprinsäuretriglycerid | 4,0 |
| Isopropylmyristat | 4,0 |
| Erbsenproteinhydrolysat (analog Beispiel 1) | 1,0 |

| **Wasserphase:** | |
|---|---|
| | |
| 1,2-Propandiol | 2,0 (Gew.%) |
| MgSO₄ x 7H₂O | 0,7 |
| Glycerin | 4,0 |
| Isobutylen/Maleinsäure-Copolymer (Aquaflex^{R}FX64) | 5,0 |
| Wasser ad | 100,0 |

Diese W/O-Emulsion wies ähnlich gute Eigenschaften wie diejenige nach Beispiel 4 auf.

### Beispiel 6

Analog dem in Beispiel 4 beschriebenen Verfahren wurde eine W/O-Emulsion der folgenden Zusammensetzung hergestellt:

| **Fett- bzw. Ölphase:** | |
|---|---|
| | |
| Kartoffelproteinhydrolysat (Löslichkeit in 100 ml H₂O bei 20°C:< 0,25 g) | 0,5 (Gew.%). |
| Erbsenproteinhydrolysat (Löslichkeit in 100 ml H₂O bei 20°C:< 0,3 g) | 0,5 |
| Diisostearylpolyglyceryl-3 Dimerdilinoleat (Isolan® PDI) | 2,0 |
| Lauryl Dimethicone Copolyol | 2,0 |
| Triglycerindiisostearat | 1,5 |
| Isostearinsäurediethanolamid | 1,5 |
| 2-Octyldodecanol | 2,0 |
| Vaseline | 12,0 |
| Capryl-/Caprinsäuretriglycerid | 4,0 |
| Isopropylstearat | 3,0 |

| **Wasserphase:** | |
|---|---|
| | |
| 1,2-Propandiol | 3,0 (Gew.%) |
| MgSO₄ x 7H₂O | 0,7 |
| Glycerin | 3,0 |
| N-Octylacrylamid/Acrylsäure/ | 2,5 |
| N-tert. Butylaminoethylmethacrylat-Terpolymer (Amphomer^{R}) | 1,0 |
| Polyvinylpyrrolidon | 0,5 |
| Aminomethylpropandiol | q.s. |
| Konservierungsmittel | q.s. |
| Wasser ad | 100,0 |

Es wurde ein stabiles Produkt mit ausgezeichneten haarkonditionierenden Eigenschaften erhalten.

## Patentansprüche

1. Haarnachbehandlungsmittel, bestehend aus einer Wasser-in-Öl-Emulsion, **dadurch gekennzeichnet, daß** die wäßrige Phase der Emulsion mindestens ein wasserlösliches Polymer und die Öl- bzw. Fettphase der Emulsion mindestens ein Pflanzenproteinhydrolysat, dessen Klarlöslichkeit im Wasser bei 20°C weniger als 0,5g/100 ml H₂O, vorzugsweise weniger als 0,3g/100 ml H₂O beträgt, enthält.

2. Mittel nach Anspruch 1, enthaltend 0,25 bis 7,5 Gew.-%, berechnet auf die Gesamtzusammensetzung, mindestens eines wasserlöslichen Polymeren.

3. Mittel nach Anspruch 1 oder 2, enthaltend 0,1 bis 7,5 Gew.-%, berechnet auf die Gesamtzusammensetzung, mindestens eines Pflanzenproteinhydrolysats, dessen Klarlöslichkeit im Wasser bei 20°C weniger als 0,5g/100 ml H₂O, vorzugsweise weniger als 0,3g/100 ml H₂O beträgt,.

4. Mittel nach einem der Ansprüche 1 bis 3, **dadurch gekennzeichnet, daß** das Pflanzenproteinhydrolysat ein Molekulargewicht zwischen etwa 10 000 und etwa 800 000 g/Mol aufweist.

5. Mittel nach einem oder mehreren der Ansprüche 1 bis 4, **dadurch gekennzeichnet, daß** es 0,5 bis 25 Gew.-%, berechnet auf die Gesamtzusammensetzung, mindestens eines C₈-C₂₀-Alkyl Dimethicone Copolyols der allgemeinen Formel 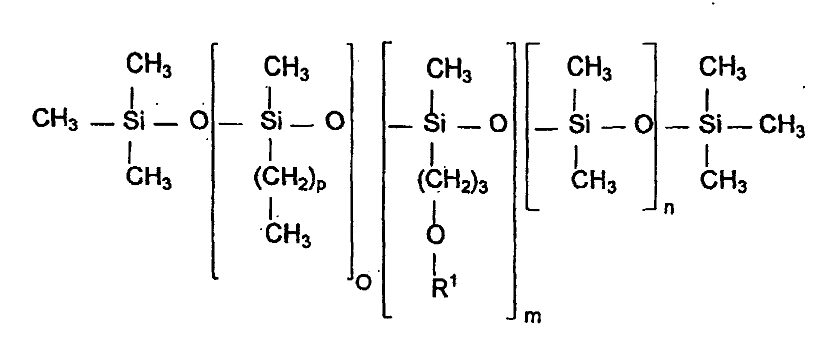 worin R¹ eine Polyethergruppe mit 1-60 Ethylenoxid- und/oder Propylenoxideinheiten, m, n und o je eine Zahl von 1 bis 500, und p eine Zahl von 7 bis 19 Kohlenstoffatomen bedeuten und/oder eines Di-C₁₂-C₂₀-acylpolyglyceryl-2-10 Dimer-dioleats und/oder - dilinoleats enthält.

6. Mittel nach Anspruch 5, enthaltend als Alkyl Dimethicone Copolyol Lauryl und/oder Cetyl Dimethicone Copolyol.

7. Mittel nach Anspruch 5, enthaltend als Di-C₁₂-C₂₀-acylpolyglyceryl-2-10 Dimerdilinoleat Diisostearylpolyglyceryl-3 Dimerdilinoleat.

## Claims

1. Hair after-treatment composition, consisting of a water-in-oil emulsion, wherein the aqueous phase of the emulsion contains at least one water-soluble polymer and the oily or fatty phase of the emulsion contains at least one plant protein hydrolyzate which transparent solubility in water by a temperature of 20°C is less than 0,5g/100 ml H₂O, preferably less than 0,3g/100 ml H₂O.

2. Composition according to claim 1, containing 0.25 % to 7.5 % by weight, calculated to the total composition, of at least one water-soluble polymer.

3. Composition according to claim 1 or 2, containing 0.1 % to 7.5 % by weight, calculated to the total composition, of at least one plant protein hydrolyzate which transparent solubility in water by a temperature of 20°C is less than 0,5g/100 ml H₂O, preferably less than 0,3g/100 ml H₂O.

4. Composition according to one or more of claims 1 to 3, wherein the plant protein hydrolyzate has a molecular weight ranging between about 10,000 and about 800,000 g/mol.

5. Composition according to one or more of claims 1 to 4 **characterized in that** it comprises 0.5 % to 25 % by weight, calculated to the total composition, of at least one C₈-C₂₀-alkyl dimethicone copolyol of the general formula 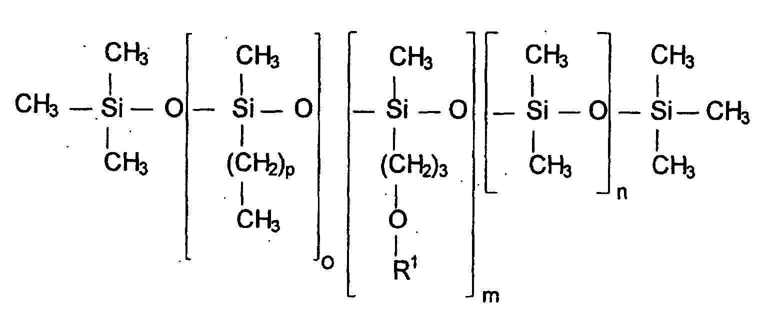 wherein R¹ is a polyether group with 1-60 ethyleneoxide and/or propyleneoxide units, m, n and o each are a number from 1 to 500, and p is a number from 7 to 19 carbon atoms and/or one di-C₁₂-C₂₀-acyl polyglyceryl-2-10 dimerdioleate and/or dilinoleate.

6. Composition according to claim 5, comprising as alkyl dimethicone copolyol lauryl and/or cetyl dimethicone copolyol.

7. Composition according to claim 5, comprising as di-C₁₂-C₂₀-acyl polyglyceryl-2-10 dimerdilinoleate diisostearyl polyglyceryl-3 dimerdilinoleate.

## Revendications

1. Agent de post-traitement capillaire, constitué d'une émulsion eau-dans-huile, **caractérisé en ce que** la phase aqueuse de l'émulsion contient au moins un polymère hydrosoluble et la phase d'huile ou de matière grasse contient un hydrolysat de protéines végétales, dont la solubilité jusqu'à transparence dans l'eau à 20°C est moins de 0,5g/100 ml de H₂O, de préférence moins de 0,3 g/100 ml de H₂O.

2. Agent selon la revendication 1, contenant 0,25 à 7,5% en poids, calculé par rapport à la composition totale, d'au moins un polymère hydrosoluble.

3. Agent selon la revendication 1 ou 2, contenant 0,1 à 7,5% en poids, calculé par rapport à la composition totale, d'au moins un hydrolysat de protéines végétales dont la solubilité jusqu'à transparence dans l'eau à 20°C est moins de 0,5g/100 ml de H₂O, de préférence moins de 0,3 g/100 ml de H₂O.

4. Agent selon l'une quelconque des revendications 1 à 3, **caractérisé en ce que** l'hydrolysat de protéines végétales présente une masse moléculaire comprise entre environ 10 000 et 800 000 g/mole.

5. Agent selon l'une ou plusieurs des revendications 1 à 4, **caractérisé en ce qu'**il contient 0,6 à 25% en poids, calculé par rapport à la composition totale, d'au moins un copolyol de (alkyle en C₈ à C₂₀)-diméthicone de formule générale 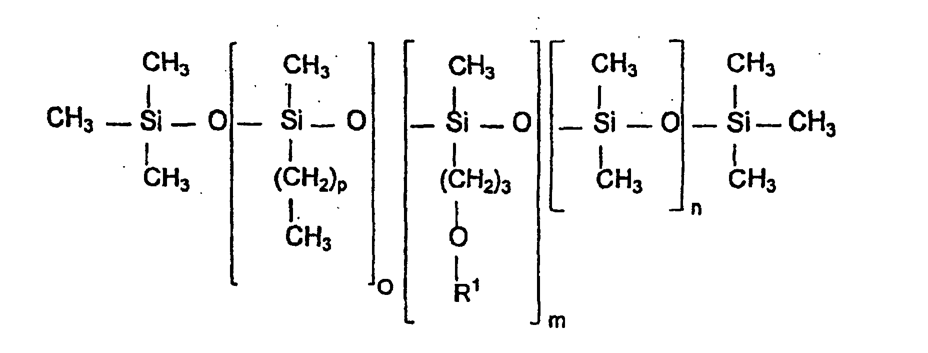 dans laquelle R¹ représente un groupe polyéther ayant 1 à 60 motifs d'oxyde d'éthylène et/ou d'oxyde de propylène, m, n et o représentent chacun un nombre de 1 à 500, et p représente un nombre de 7 à 19 atomes de carbone, et/ou d'un di-(acyle en C₁₂ à C₂₀)-polyglycéryl-2-10-dioléate et/ou di-(acyle en C₁₂ à C₂₀)-polyglycéryl-2-10-dilinoléate dimère.

6. Agent selon la revendication 5, contenant un copolyol de lauryldiméthicone et/ou de cétyldiméthicone en tant que copolyol d'alkyldiméthicone.

7. Agent selon la revendication 5, contenant un di-isostéarylpolyglycéryl-3-dilinoléate dimère en tant que di-(acyle en C₁₂ à C₂₀)-polyglycéryl-2-10-dilinoléate dimère.
